# EUROPÄISCHE PATENTSCHRIFT

(11) **EP 0 623 323 B1**
(45) Veröffentlichungstag und Bekanntmachung des Hinweises auf die Patenterteilung: **29.05.1996**
(21) Anmeldenummer: 94110049.7
(22) Anmeldetag: 04.12.1991
(51) Int. Cl.: A61F 2/02

(54) **Verfahren zur Herstellung eines medizinischen Implantats und so hergestelltes Implantat**
Process for preparing a medical implant and implant prepared according to the process
Procédé de préparation d'un implant médical et l'implant médical préparé selon ledit procédé

(30) Priorität: 19.12.1990 DE 4040581; 19.01.1991 DE 4101526
(43) Veröffentlichungstag der Anmeldung: 09.11.1994
(62) Teilanmeldung aus: 91120802.3
(73) Patentinhaber: Härle, Anton, Prof. Dr., D-48161 Münster (DE)
(72) Erfinder: Härle, Anton, Prof. Dr., D-48161 Münster (DE)

(56) Entgegenhaltungen:
- EP-A- 0 353 476
- EP-A- 0 398 497
- WO-A-87/06842
- WO-A-87/07827
- WO-A-88/01517
- WO-A-89/09035
- WO-A-90/00037
- US-A- 4 643 735

## Beschreibung

Die Erfindung bezieht sich auf ein Verfahren zur Herstellung medizinischer Implantate für den Einsatz in der Human- und Tiermedizin und ein so hergestelltes Implantat.

Die rekonstruktiven, medizinischen Behandlungsverfahren erfordern in zunehmendem Maße Ersatzmaterialien, z.B. für Knochengewebe, um die formgerechte Wiederherstellung bzw. den Aufbau einer anatomischen Form von Knochen, Knochenanteilen und anderen Gewebsabschnitten erreichen zu können, da die Verwendung von körpereigenem Gewebe z.B. Knochen häufig an die Grenze der ausreichenden Verfügbarkeit stößt und auch mit der Problematik der Zweiterkrankheit am Entnahmeort belastet ist.

So hat z.B. der Einsatz von der Knochensubstanz ähnlichen Hydroxylapatit-Keramikmaterial in den letzten 20 Jahren großes wissenschaftliches Interesse gefunden, aber nicht zu dem erwarteten Erfolg geführt. Zwar ist gelungen, sehr verschiedene Biokeramikmaterialien künstlich mit unterschiedlicher Löslichkeit und Resorptionseigenschaften herzustellen, doch konnten bisher mit Ausnahme weniger Anwendungsgebiete Hydroxylapatitkeramiken herkömmlicher Ausgestaltung die autologe Knochentransplantation nicht ersetzen, nicht einmal mengenmäßig einschränken.

Die Gründe dafür liegen einerseits darin, daß die bisherigen Implantate entweder keine ausreichende Stabilität im Sinne einer mechanischen Beanspruchbarkeit aufweisen oder, wenn sie diese Eigenschaft haben, nicht in Körpergewebe integriert werden, d.h. eine unzureichende Biodegradibilität besitzen. Neben der Biodegrabilität ist aber die Integration in das Umgebungsgewebe auch davon abhängig, ob z.B. im Implantatlager das Auftreten von Entzündungen verhindert werden kann. Zu diesem Zwecke müßten die Implantate z.B. eine eigene pharmakologische und diesem Fall antizündliche Wirkung entfalten. Bisherige Implantate wurden zwar in sterilisierten Zustand angeliefert, konnten aber keine eigene pharmakologische Wirkung im Gewebe entfalten, da sie in der Regel keine Pharmaka enthielten und auch keine Vorkehrung für eine Depothaltung und eine Freisetzung im Gewebe beinhalteten.

In der EPA 0398 497 wird ein Verfahren zur individuellen Formgebung von Implantatkörpern beschrieben, die aus einzelnen Partikeln hergestellt werden, wobei zur Aufrechterhaltung der gewünschten individuellen Form die Einzelpartikel in einem flexiblen Beutel durch Unterdruck aneinander fixiert werden. Durch den nachfolgenden Sinterungs- oder Polymerisationsprozeß entsteht dann der individuell geformte poröse Implantatkörper. Über eine weitere Anwendung oder Aufrechterhaltung eines Unterdrucks im hergestellten Implantatkörper, insbesondere zum Zwecke der Befüllung mit Pharmaka, gibt diese Druckschrift keine Hinweise.

Demgegenüber liegt der Erfindung die Aufgabe zugrunde, ein Herstellungsverfahren für Implantate zu offenbaren, das diese Implantate entweder in einem Zustand bereitstellt, der die Befüllung der von Poren gebildeten Hohlräume mit Pharmaka ermöglicht, oder die Implantate in einen derartigen Zustand überführen läßt.

Diese der Erfindung zugrunde liegende Aufgabe, nämlich der Ausbildung der Implantate dergestalt, daß sie zur optimalen Integration in das Implantatlager mit Pharmaka angereichert werden können, wird durch die Lehre des Hauptanspruches gelöst. Vorteilhafte Ausgestaltungen sind in den Unteransprüchen erläutert.

Nach der erfindungsgemäßen Verfahrensweise werden Implantate mit an die Oberfläche des Implantats reichenden Mikro- und/oder Makroporen ausgestattet und die von den Poren gebildeten Hohlräume in einem Behälter evakuiert, sodaß sie einen unter dem atmosphärischen Außendruck liegenden Gasdruck aufweisen.

Beispielsweise können die Implantate in evakuierten und luftdichten Verpackungen eingeschlossen und in dieser Weise gelagert sein, so daß das unter Vakuum stehende Implantat an den Arzt oder Krankenhaus ausgeliefert wird. Vorzugsweise wird die Verpackung aus einem Verpackungsbehälter bestehen. Der Arzt kann nunmehr in den, dieses evakuierte Implantat enthaltenden, Verpackungsbehälter ein oder mehrere Pharmaka einbringen, die sich dann auf der Oberfläche und an die inneren Oberflächen der Implantate anlagern bzw. in diese aufgenommen werden und dann nach dem Einbringen des Implantats in den menschlichen oder tierischen Körper freigesetzt werden.

Vorzugsweise liegt der Gasdruck im Verpackungsbehälter niedriger als 0,3 bar.

Hierbei ist wichtig, daß diese Verpackung aus einem luftdicht verschließbaren Behälter besteht, der starrwandig oder flexibelwandig ausgebildet ist, aber evakuierbar ist.

Vorzugsweise beinhaltet der Verpackungsbehälter eine Zugangsmöglichkeit, die entweder aus einer Durchstechmembrane oder aus einem Zugangsventil besteht. Durch die Zugangsmöglichkeit kann nunmehr der behandelnde Arzt Zugang zum Inneren des Behälters erlangen, ohne daß dadurch das Vakuum im Behälter gestört wird und kann in von Poren gebildeten Hohlräume des Implantates Pharmaka einbringen.

Zur Verdeutlichung des Erfindungsgedankens werden in der Zeichnung die Formkörper in zwei unterschiedlichen Ausgestaltungen dargestellt.

Die Zeichnung zeigt in
- Fig. 1: in Draufsicht, eine Ansicht von unten und im Schnitt ein Implantat, in
- Fig. 2: das in Fig. 1 dargestellte Implantat, wobei die Hohlräume mit der zweiten Materialkomponente ausgefüllt ist.

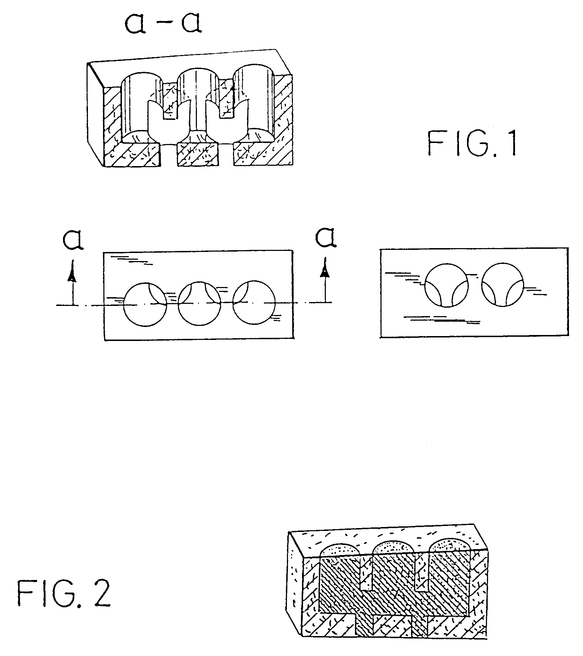

Die in den Fig. 1 und 2 dargestellten Formkörper sind leicht keilförmig mit einem Neigungswinkel α zwischen 2 und 19° ausgebildet und können z.B. aus zwei Materialkomponenten, von denen die erste Materialkomponente eine hohe mechanische Festigkeit und die zweite eine mikroporöse Struktur und gute biologische Integrationsfähigkeit mittels großer spezifischer Oberfläche besitzt.

Die Zeichnungen verdeutlichen die Möglichkeit des Anbringens der Makroporen (= Öffnungen), wobei z.B. die Seitenflächen von Öffnungen frei sein können.

In der Fig. 2 ist dargestellt, wie die zweite, die Integration fördernde Materialkomponente in die Hohlräume der ersten Materialkomponente eingebracht ist.

Diese zweite Materialkomponente kann nunmehr mikroporös ausgebildet sein. Wird ein solcher poröser Implantatkörper evakuiert und in evakuierten Zustand verpackt z.B. an den Operateur ausgeliefert, ist es möglich, nunmehr durch Injektion in die unter Vakuum stehende Verpackung in den porösen Implantatkörper "Pharmaka" einzuspritzen, die begierig von den Poren aufgenommen werden, so daß dann, wenn diese Implantatkörper implantiert werden, im Körper diese Pharmaka freigegeben und freigesetzt werden können.

## Patentansprüche

1. Verfahren zur Herstellung eines Implantats für den Einsatz in der Human- und Tiermedizin, dadurch gekennzeichnet, daß das Implantat mit an die Oberfläche des Implantats reichenden Mikro- und/oder Makroporen ausgestattet wird und die von den Poren gebildeten Hohlräume in einem Behälter evakuiert werden, so daß sie einen unter dem atmosphärischen Außendruck liegenden Gasdruck aufweisen.

2. Verfahren nach Anspruch 1, dadurch gekennzeichnet, daß das Implantat im evakuierten Zustand innerhalb eines Verpackungsbehälter angeordnet wird.

3. Verfahren nach Anspruch 2, dadurch gekennzeichnet, daß der Gasdruck im das Implantat enthaltenden Verpackungsbehälter niedriger als 0,3 bar eingestellt wird.

4. Implantat, hergestellt nach einem oder mehreren der vorangehenden Ansprüche, dadurch gekennzeichnet, daß der Gasdruck in den Hohlräumen des Implantatkörpers niedriger als 0,3 bar ist.

5. Verfahren nach einem oder mehreren der vorangehenden Ansprüche, dadurch gekennzeichnet, daß der Behälter oder Verpackungsbehälter luftdicht verschließbar ist.

6. Verfahren nach Anspruch 5, dadurch gekennzeichnet, daß der Behälter oder Verpackungsbehälter mit einer Durchstechmembrane und/oder einem Zugangsventil ausgerüstet ist.

7. Implantat nach Anspruch 4 in Kombination mit einer Verpackung, dadurch gekennzeichnet, daß der Behälter luftdicht verschließbar ist und mit einer Durchstechmembrane und/oder einem Zugangsventil ausgerüstet ist.

## Claims

1. A process for producing an implant for use in human and veterinary medicine, characterized in that the implant is provided with micro- and/or macro-pores extending to the surface of the implant and the cavities formed by the pores are evacuated in a container, such that they exhibit a gas pressure below external atmospheric pressure.

2. A process according to claim 1, characterized in that the implant is arranged inside a packaging container in the evacuated state.

3. A process according to claim 2, characterized in that the gas pressure in the packaging container containing the implant is set lower than 0.3 bar.

4. An implant, produced according to any one of the preceding claims, characterized in that the gas pressure in the cavities in the implant member is lower than 0.3 bar.

5. A process according to any one of the preceding claims, characterized in that the container or packaging container is air-tightly sealable.

6. A process according to claim 5, characterized in that the container or packaging container is provided with a perforatable membrane and/or an access valve.

7. An implant according to claim 4 combined with packaging, characterized in that the container is air-tightly sealable and provided with a perforatable membrane and/or an access valve.

## Revendications

1. Procédé de préparation d'un implant pour une application en médecine humaine et vétérinaire, caractérisé en ce que l'implant est muni de micropores et/ou de macropores atteignant la surface de l'implant et que l'air est évacué des cavités créés par les pores dans un récipient de sorte à présenter une pression gazeuse inférieure à la pression atmosphérique extérieure.

2. Procédé selon la revendication 1, caractérisé en ce que l'implant est placé sous état de vide à l'intérieur d'un récipient de conditionnement.

3. Procédé selon la revendication 2, caractérisé en ce que la pression gazeuse dans le récipient de conditionnement contenant l'implant est réglée sur moins de 0,3 bar.

4. Implant, préparé selon une ou plusieurs des revendications précédantes, caractérisé en ce que la pression gazeuse dans les cavités du corps d'implant est inférieure à 0,3 bar.

5. Procédé selon une ou plusieures des revendications précédantes, caractérisé en ce que le récipient ou le récipient de conditionnment peut être hermétiquement scellé.

6. Procédé selon revendication 5, caractérisé en ce que le récipient ou récipient de conditionnement est muni d'une membrane perforble et/ou d'une soupape d'accès.

7. Implant selon la revendication 4 en combinaison avec un emballage, caractérisé en ce que le récipient peut être hermétiquement scellé et qu'il est muni d'une membrane perforable et/ou d'une soupape d'accès.
